(19) Europäisches Patentamt / European Patent Office / Office européen des brevets

(11) **EP 4 691 639 A1**

(12) **EUROPEAN PATENT APPLICATION**
published in accordance with Art. 153(4) EPC

(43) Date of publication:
**11.02.2026   Bulletin 2026/07**

(21) Application number: **24779878.8**

(22) Date of filing: **21.03.2024**

(51) International Patent Classification (IPC):
**B01J 35/57** (2024.01)   **B01J 23/80** (2006.01)
**C07B 61/00** (2006.01)   **C07C 1/12** (2006.01)
**C07C 9/04** (2006.01)

(52) Cooperative Patent Classification (CPC):
**B01J 23/80; B01J 35/57; C07B 61/00; C07C 1/12;
C07C 9/04**

(86) International application number:
**PCT/JP2024/011126**

(87) International publication number:
**WO 2024/203759 (03.10.2024 Gazette 2024/40)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **31.03.2023   JP 2023057141**

(71) Applicant: **NGK Insulators, Ltd.
Nagoya-shi, Aichi 467-8530 (JP)**

(72) Inventors:
• **MAEDA Kazuki**
  **Nagoya-shi, Aichi 467-8530 (JP)**
• **HORI Keigo**
  **Nagoya-shi, Aichi 467-8530 (JP)**
• **OKADA MUKOGAWA Akane**
  **Nagoya-shi, Aichi 467-8530 (JP)**
• **KOBAYASHI Yoshimasa**
  **Nagoya-shi, Aichi 467-8530 (JP)**

(74) Representative: **Müller-Boré & Partner
Patentanwälte PartG mbB
Friedenheimer Brücke 21
80639 München (DE)**

(54) **HONEYCOMB STRUCTURE**

(57)   Provided is a honeycomb structural body that enables production of methane in an energy-saving manner at an excellent methane conversion rate. A honeycomb structural body according to an embodiment of the present disclosure includes a honeycomb-like base material and catalyst layers. The honeycomb-like base material includes a partition wall that defines a plurality of cells. The cells each include a gas flow passage. The catalyst layers contain a methanation reaction catalyst for methanation of carbon dioxide through a reaction with hydrogen. The catalyst layers have a difference in the amount of the methanation reaction catalyst supported per unit area of the partition wall in a direction of passage of a fluid through the gas flow passages.

FIG. 1

## Description

Technical Field

The present disclosure relates to a honeycomb structural body.

Background Art

In recent years, capture of carbon dioxide and its reuse as a raw material for a carbon compound have been under consideration in terms of a reduction in environmental load. For example, methanation for converting carbon dioxide to methane through a reaction with hydrogen has been proposed (see, for example, Patent Literature 1). In such methanation, a raw material gas containing carbon dioxide and hydrogen is supplied to a reactor that has been heated at a predetermined temperature so that a methanation reaction progresses. However, the methanation reaction is an exothermic reaction, and a temperature rises in the reactor portion in which the methanation reaction has occurred. Meanwhile, chemical equilibrium in the methanation reaction under normal pressure is reduced when the temperature exceeds 450°C. Thus, when a reaction and temperature control are not properly performed in accordance with a location in the reactor, a methane conversion rate may sometimes be insufficient.

Citation List

Patent Literature

[PTL 1] JP 2015-196619 A

Summary of Invention

Technical Problem

A primary object of the present disclosure is to provide a honeycomb structural body that enables production of methane in an energy-saving manner at an excellent methane conversion rate.

Solution to Problem

[1] According to one embodiment of the present disclosure, there is provided a honeycomb structural body including a honeycomb-like base material and catalyst layers. The honeycomb-like base material includes a partition wall that defines a plurality of cells. The cells each include a gas flow passage. The catalyst layers contain a methanation reaction catalyst for methanation of carbon dioxide through a reaction with hydrogen. The catalyst layers are each provided on a surface of the partition wall so as to face the gas flow passages, respectively. The catalyst layers have a difference in the amount of the methanation reaction catalyst supported per unit area of the partition wall in a direction of passage of a fluid through the gas flow passages.

[2] In the honeycomb structural body according to the above-mentioned item [1], the supported amount of the methanation reaction catalyst may increase toward a downstream side in the passage direction.

[3] In the honeycomb structural body according to the above-mentioned item [2], the catalyst layers may each have a first end portion being an upstream end portion in the passage direction, and a second end portion being a downstream end portion in the passage direction. When an overall length of each of the catalyst layers in the passage direction is defined as 100% and the first end portion of the catalyst layer is defined as 0%, the supported amount of the methanation reaction catalyst in a range of from 0% to 10% of the catalyst layer may be smaller than the supported amount of the methanation reaction catalyst in a range of from 50% to 100% of the catalyst layer.

[4] In the honeycomb structural body according to the above-mentioned item [2] or [3], when an overall length of each of the catalyst layers in the passage direction is defined as 100% and a first end portion of the catalyst layer is defined as 0%, a thickness of the catalyst layer at a position corresponding to 50% may be larger than a thickness of the catalyst layer at a position corresponding to 5%.

[5] In the honeycomb structural body according to the above-mentioned item [4], the catalyst layers may each include a thin portion and a thick portion in the stated order in the passage direction.

[6] In the honeycomb structural body according to any one of the above-mentioned items [1] to [5], the methanation reaction catalyst may contain Ni as an active component, and a cerium oxide carrier for supporting Ni.

Advantageous Effects of Invention

According to the embodiment of the present disclosure, the honeycomb structural body that enables production of methane in an energy-saving manner at an excellent methane conversion rate can be achieved.

Brief Description of Drawings

FIG. **1** is a schematic perspective view of a honeycomb structural body according to one embodiment of the present disclosure.

FIG. **2** is a schematic sectional view of the honeycomb structural body of FIG. **1**.

FIG. **3** is a schematic sectional view of a honeycomb structural body according to another embodiment of the present disclosure.

Description of Embodiments

Embodiments of the present disclosure are described below with reference to the drawings. However, the present disclosure is not limited to these embodiments. In addition, in the drawings, the width, thickness, shape, and the like of each portion may be schematically illustrated as compared to those in the embodiments in order to provide clearer description, but the drawings are merely examples and do not limit the interpretation of the present disclosure.

A. Overview of Honeycomb Structural Body

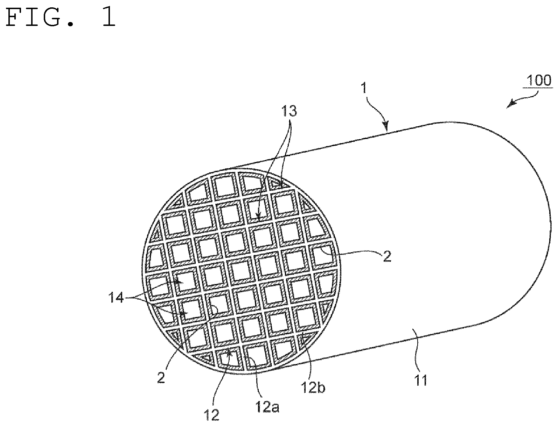

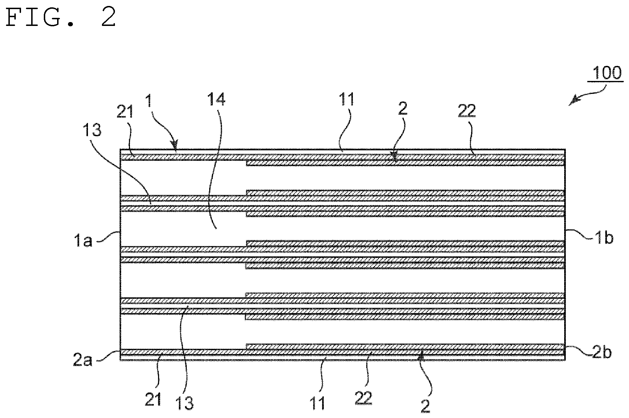

FIG. **1** is a schematic perspective view of a honeycomb structural body according to one embodiment of the present disclosure, and FIG. **2** is a schematic sectional view of the honeycomb structural body of FIG. **1**.

A honeycomb structural body **100** of the illustrated example includes a honeycomb-like base material **1** and catalyst layers **2**. The honeycomb-like base material **1** includes a partition wall **12** that defines a plurality of cells **13**. The cells **13** each include a gas flow passage **14**. The catalyst layers **2** contain a methanation reaction catalyst for methanation of carbon dioxide through a reaction with hydrogen. The catalyst layers **2** are formed on a surface of the partition wall **12** so as to face the gas flow passages **14,** respectively. The catalyst layers **2** have a difference in the amount of the methanation reaction catalyst supported per unit area of the partition wall **12** (hereinafter referred to as "supported catalyst amount") in a direction of passage of a fluid through the gas flow passages **14** (hereinafter referred to as "passage direction").

In the configuration as described above, the catalyst layers having a difference in the supported catalyst amount in the passage direction are provided on the surface of the partition wall of the honeycomb-like base material. Thus, when a raw material gas containing at least carbon dioxide and hydrogen is caused to flow through the gas flow passages to be supplied to the catalyst layers, carbon dioxide and hydrogen can be appropriately brought into contact with the methanation reaction catalyst in accordance with a composition of the raw material gas. Accordingly, a reaction and temperature control can be properly adjusted in accordance with a location in the honeycomb structural body without leading to complication of a methanation production apparatus and/or an increase in size thereof, and hence a methanation reaction expressed by the following Formula (1) is allowed to smoothly progress. The methanation reaction (1) is an exothermic reaction. Thus, generated heat (reaction heat) can be effectively utilized for the progress of a chemical reaction. As a result, methane can be produced in an energy-saving manner at an excellent methane conversion rate.

$$CO_2 + 4H_2 \rightarrow CH_4 + 2H_2O \qquad (1)$$

In one embodiment, the supported catalyst amount increases toward a downstream side in the passage direction. The supported catalyst amount may increase continuously or in a stepwise manner toward the downstream side in the passage direction.

The raw material gas sometimes contains oxygen in addition to carbon dioxide and hydrogen, which is described later in detail. In this case, when the raw material gas is supplied to the catalyst layers, and hydrogen and oxygen are brought into contact with the methanation reaction catalyst, a combustion reaction expressed by the following Formula (2) can progress. The combustion reaction (2) is an exothermic reaction. Thus, generated reaction heat can be effectively used for the progress of the above-mentioned methanation reaction (1).

$$O_2 + 2H_2 \rightarrow 2H_2O \qquad (2)$$

Meanwhile, the above-mentioned combustion reaction (2) has a reaction speed faster than that of the above-mentioned methanation reaction (1), and may progress preferentially in an upstream-side part of the catalyst layers in the passage direction. Then, a localized high temperature area (hot spot) is sometimes formed in the upstream-side part of the catalyst layers in the passage direction. The formation of a hot spot may lower the methane conversion rate and/or may degrade the methanation reaction catalyst.

On the other hand, in one embodiment, the supported catalyst amount increases toward the downstream side in the passage direction. Thus, the formation of a hot spot in the upstream-side part of the catalyst layers can be prevented, and the above-mentioned methanation reaction (1) is allowed to sufficiently progress in a downstream-side part of the catalyst layers. Thus, the improvement in the methane conversion rate and a longer life of the methanation reaction catalyst can be achieved.

The catalyst layers **2** each typically have a first end portion **2a** and a second end portion **2b** (see FIG. **2**). The first end portion **2a** is an upstream end portion in the passage direction, and the second end portion **2b** is a downstream end portion in the passage direction.

In one embodiment, when an overall length of the catalyst layer **2** in the passage direction is defined as 100%, and the first end portion **2a** of the catalyst layer **2** is defined as 0%, the supported catalyst amount in a range of from 0% to 10% of the catalyst layer **2** is smaller than the supported catalyst amount in a range of from 50% to 100% of the catalyst layer **2.** Thus, the formation of a hot spot in the upstream-side part (range of from 0% to 10%) of the catalyst layer can be stably prevented.

The supported catalyst amount in the range of from 0% to 10% of the catalyst layer **2** is, for example, from 1.0 mg/mm$^2$ to 6.0 mg/mm$^2$, preferably from 2.0 mg/mm$^2$ to 5.0 mg/mm$^2$. When the supported catalyst amount in the above-mentioned range falls within such ranges, the methanation reaction (1) and the combustion reaction (2), which are described above, are allowed to progress in a well-balanced manner. Thus, energy savings can be stably achieved, and the methane conversion rate can be improved.

The supported catalyst amount in the range of from 50% to 100% of the catalyst layer **2** is, for example, from 5.0 mg/mm$^2$ to 20 mg/mm$^2$, preferably from 6.0 mg/mm$^2$ to 16.0 mg/mm$^2$. When the supported catalyst amount in the above-mentioned range falls within such ranges, the above-mentioned methanation reaction (1) is allowed to more smoothly progress in the downstream-side part of the catalyst layers to thereby further improve the methane conversion rate.

The supported catalyst amount in a range of from more than 10% to less than 50% (hereinafter referred to as "from 10% to 50%") of the catalyst layer **2** may be appropriately and suitably adjusted in accordance with applications. For example, the supported catalyst amount in the range of from 10% to 50% of the catalyst layer **2** may be the same as the supported catalyst amount in the above-mentioned range of from 0% to 10% or may be the same as the supported catalyst amount in the above-mentioned range of from 50% to 100%. The supported catalyst amount in the range of from 10% to 50% of the catalyst layer **2** is specifically from 1.0 mg/mm$^2$ to 20 mg/mm$^2$, preferably from 2.0 mg/mm$^2$ to 16 mg/mm$^2$.

A difference in the supported catalyst amount in the catalyst layers **2** can be achieved by any appropriate configuration. For example, a thickness of each of the catalyst layers **2** may be adjusted while a content ratio of the methanation reaction catalyst in the catalyst layers **2** is set substantially constant, or the content ratio of the methanation reaction catalyst in the catalyst layers **2** may be adjusted while the thickness of each of the catalyst layers **2** is set substantially constant.

In one embodiment, the thickness of the catalyst layer **2** is appropriately adjusted while the content ratio of the methanation reaction catalyst in the catalyst layers **2** is set substantially constant. Typically, when the overall length of the catalyst layer **2** in the passage direction is defined as 100%, and the first end portion **2a** of the catalyst layer **2** is defined as 0%, the thickness of the catalyst layer **2** at a position corresponding to 50% is larger than the thickness of the catalyst layer **2** at a position corresponding to 5%.

The thickness of the catalyst layer **2** at the position corresponding to 50% is, for example, 1.2 times or more, preferably 1.5 times or more, more preferably 2.0 times or more the thickness of the catalyst layer **2** at the position corresponding to 5%. Meanwhile, the thickness of the catalyst layer **2** at the position corresponding to 50% is, for example, 5.0 times or less, preferably 4.0 times or less the thickness of the catalyst layer **2** at the position corresponding to 5%. When the thickness of the catalyst layer at the position corresponding to 50% falls within such ranges, the methane conversion rate can be further improved.

In the illustrated example, the catalyst layer **2** includes a thin portion **21** and a thick portion **22** in the stated order in the passage direction.

The thin portion **21** is positioned on an upstream side with respect to the thick portion **22** in the passage direction. The thick portion **22** is positioned on a downstream side with respect to the thin portion **21** in the passage direction.

Typically, the thin portion **21** is positioned at least within the range of from 0% to 10% of the catalyst layer **2,** and the thick portion **22** is positioned at least within the range of from 50% to 100% of the catalyst layer **2.** In a range of from more than 10% to less than 50% (hereinafter referred to as "from 10% to 50%") of the catalyst layer **2,** any of the thin portion **21** or the thick portion **22** may be positioned.

In other words, the range of the thin portion **21** in the catalyst layer **2** is from 0% to (50-$\alpha$)%, and the range of the thick portion **22** in the catalyst layer **2** is from (50-$\alpha$)% to 100%. The symbol $\alpha$ represents, for example, 0 or more and 45 or less, for example, 0 or more and 40 or less, for example, 0 or more and 30 or less, for example, 0 or more and 20 or less, and, for example, 0 or more and 10 or less.

The range of the thin portion **21** in the catalyst layer **2** is, for example, from 0% to 10% of the catalyst layer **2,** and, for example, from 0% to 5% of the catalyst layer **2.** The thin portion **21** is thinner than the thick portion **22.** A thickness of the thin portion **21** is, for example, from 1 $\mu$m to 20 $\mu$m, preferably from 5 $\mu$m to 15 um.

The range of the thick portion **22** in the catalyst layer **2** is, for example, from 50% to 100% of the catalyst layer **2,** for example, from 20% to 100% of the catalyst layer **2,** for example, from 10% to 100% of the catalyst layer **2,** and, for example, from 5% to 100% of the catalyst layer **2.** A thickness of the thick portion **22** is, for example, from 10 um to 80 $\mu$m, preferably from 20 $\mu$m to 50 $\mu$m, more preferably from 25 $\mu$m to 35 $\mu$m. A range of a thickness ratio of the thick portion **22** to the thin portion **21** is, for example, the same as the range of the above-mentioned thickness ratio of the thickness of the catalyst layer **2** at the position corresponding to 50% to the thickness at the position corresponding to 5%.

In one embodiment, the content ratio of the methanation reaction catalyst in the catalyst layers **2** is substantially constant throughout the catalyst layers **2.** The content ratio of the methanation reaction catalyst in the catalyst layers **2** is, for example, 50 mass% or more, preferably 70 mass% or more, more preferably 80 mass% or more, still more preferably 90 mass% or more. Meanwhile, the upper limit of the content ratio of the methanation reaction catalyst in the catalyst layers **2** is typically 100 mass%. When the content ratio of the methanation reaction catalyst in the catalyst layers falls within such ranges, the methanation reaction can be more stably promoted.

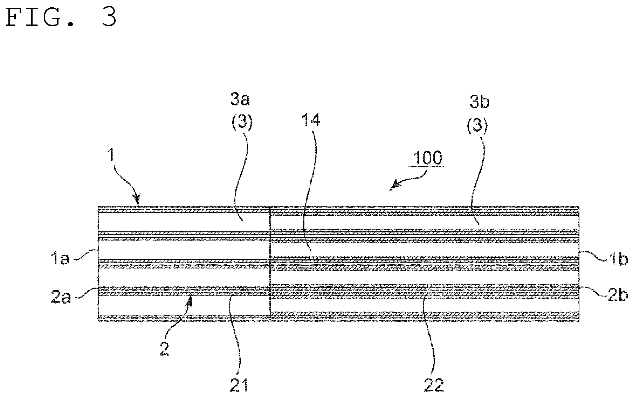

As illustrated in FIG. **3,** the honeycomb structural body **100** may be divided into a plurality of blocks **3** in the passage direction. The honeycomb structural body **100** includes a first block **3a** and a second block **3b.** The first block **3a** is positioned on an upstream side with respect to the second block **3b** in the passage direction.

The thin portions **21** are formed on the surface of the partition wall **12** in the first block **3a.** The thick portions **22** are formed on the surface of the partition wall **12** in the second block **3b.** Also with this arrangement, the catalyst layers **2** can have a difference in the supported catalyst amount in the passage direction.


B. Details of Honeycomb-like Base Material

As illustrated in FIG. **1** and FIG. **2,** the honeycomb-like base material **1** typically includes a flow-through type honeycomb structure. The honeycomb-like base material **1** has any appropriate shape (overall shape). The shape of the honeycomb-like base material **1** is, for example, a cylinder with a circle as its bottom, an elliptic cylinder with an ellipse as its bottom, a prismatic column with a polygon as its bottom, or a column with an indefinite shape as its bottom. In one embodiment, the honeycomb-like base material **1** has a columnar shape. The outer diameter and length of the honeycomb-like base material **1** may be appropriately set in accordance with purposes. The honeycomb-like base material **1** may have a hollow region in a center portion thereof in the cross section in the direction perpendicular to the lengthwise direction, though the hollow region is not shown.

In the illustrated example, the honeycomb-like base material 1 includes: an outer peripheral wall **11;** and a partition wall **12** positioned inside the outer peripheral wall **11.** The outer peripheral wall **11** and the partition wall **12** may be integrally formed or may be separate bodies. In the illustrated example, the outer peripheral wall **11** and the partition wall **12** are integrally formed.

The outer peripheral wall **11** has a cylindrical shape. The thickness of the outer peripheral wall **11** may be appropriately set in accordance with the application of the honeycomb structural body. The thickness of the outer peripheral wall **11** may be, for example, from 1 mm to 10 mm, or may be, for example, from 2 mm to 8 mm.

As described above, the partition wall **12** defines the plurality of cells **13.**

The cells **13** each extend from a first end surface **1a** (inflow end surface) of the honeycomb-like base material **1** to a second end surface **1b** (outflow end surface) thereof in the lengthwise direction (axial direction) of the honeycomb-like base material **1** (see FIG. **2**). The cells **13** each have any appropriate shape in a cross section in a direction perpendicular to the lengthwise direction of the honeycomb structural body **1.** The sectional shapes of the cells are each, for example, a triangle, a quadrangle, a pentagon, a hexagon, a higher polygon, a circle, or an ellipse. The sectional shapes and sizes of the cells may be all the same, or may be at least partly different. Of such sectional shapes of the cells, for example, a quadrangle is preferred, and a square or a rectangle is more preferred.

A cell density of the honeycomb-like base material **1** is, for example, 100 cpsi or more, preferably 300 cpsi or more. Meanwhile, the upper limit of the cell density of the honeycomb-like base material **1** is typically 1,000 cpsi. When the cell density falls within such range, carbon dioxide and hydrogen can be brought into contact with the catalyst layers in a more efficient manner.

The phrase "cell density of the honeycomb-like base material" as used herein refers to a cell density in a cross section in a lengthwise direction (direction in which the cells extend) of the honeycomb-like base material, and the unit "cpsi" as used herein refers to the number of cells for 6.4516 cm$^2$ (per square inch) of the cross section.

The gas flow passage **14** is defined inside the cell **13**, which is described later in detail. The gas flow passage **14** is a space defined inside the cell **13**, and like the cell **13**, extends from the first end surface **1a** (inflow end surface) to the second end surface **1b** (outflow end surface). Examples of a sectional shape of each of the gas flow passages **14** include shapes similar to those of the cell **13** described above. Of the sectional shapes, a quadrangle is preferred, and a square or a rectangle is more preferred. The sectional shapes and sizes of the gas flow passages **14** may be all the same, or may be at least partly different.

In the illustrated example, the partition wall **12** has a first partition wall **12a** and a second partition wall **12b** perpendicular to each other, and the first partition wall **12a** and the second partition wall **12b** define the plurality of cells **13**. The sectional shapes of the cells **13** are each a quadrangle except for a portion in which the first partition wall **12a** and the second partition wall **12b** are each brought into contact with the outer peripheral wall **11**. The configuration of the partition wall is not limited to the partition wall **12** described above. The partition wall may have a first partition wall extending in a radial direction and a second partition wall extending in a circumferential direction, which define a plurality of cells.

The thickness of the partition wall **12** may be appropriately and suitably set. The thickness of the partition wall **12** is typically smaller than the thickness of the outer peripheral wall **11**. The thickness of the partition wall **12** is, for example, 0.0127 mm (0.5 mil) or more, preferably 0.0254 mm (1.0 mil) or more. Meanwhile, the thickness of the partition wall **12** is, for example, 0.508 mm (20 mil) or less, preferably 0.254 (10 mil) mm or less, more preferably 0.2032 (8.0 mil) mm or less, still more preferably 0.127 (5.0 mil) mm or less. When the thickness of the partition wall falls within such ranges, the honeycomb structural body can achieve sufficient mechanical strength, and the cell density can be adjusted to fall within the above-mentioned ranges. The thickness of the partition wall is measured, for example, through sectional observation with a scanning electron microscope (SEM).

The partition wall **12** typically has a plurality of pores.

A mean pore diameter of the partition wall **12** may be appropriately set in accordance with purposes. The mean pore diameter of the partition wall **12** is, for example, 1 μm or more, preferably 5 μm or more. Meanwhile, the mean pore diameter of the partition wall **12** is, for example, 20 μm or less, preferably 15 μm or less. The mean pore diameter may be measured, for example, by mercury porosimetry.

A porosity of the partition wall **12** may be appropriately set in accordance with purposes. The porosity of the partition wall **12** is, for example, 15% or more, preferably 30% or more. Meanwhile, the porosity of the partition wall **12** is, for example, 60% or less, preferably 55% or less, more preferably 50% or less. The porosity is measured, for example, by mercury porosimetry.

When the mean pore diameter and/or the porosity of the partition wall **12** falls within such ranges, the supported catalyst amount in the partition wall can be improved.

A density of the partition wall **12** may be appropriately set in accordance with purposes. The density of the partition wall **12** is, for example, from 1.7 g/cm$^3$ to 2.8 g/cm$^3$, preferably from 1.8 g/cm$^3$ to 2.6 g/cm$^3$. The density is measured, for example, by mercury porosimetry.

A material for forming the honeycomb-like base material **1** is, for example, a ceramic material. Examples thereof include a zirconia-based material, an alumina-titanium carbidebased composite material, a Si-SiC-based composite material, aluminum nitride, aluminum oxide, silicon nitride, silicon carbide, zirconia, cordierite, and mullite.

Those ceramic materials may be used alone or in combination thereof. Of those ceramic materials, ceramic materials having heat resistance and oxidation resistance are preferred, and cordierite and a Si-SiC-based composite material are more preferred.

C. Details of Catalyst Layer

The catalyst layers **2** are formed on the surface of the partition wall **12**. In the honeycomb structural body **1**, the gas flow passage **14** is formed in a portion (typically, a center portion) in a cross section of the cell **13** in which the catalyst layer **2** is not formed. The catalyst layer **2** may be formed on the entire inner surface of the partition wall **12** (that is, so as to surround the gas flow passage **14**) as in the illustrated example, or may be formed on part of the surface of the partition wall. When the catalyst layer **2** is formed on the entire inner surface of the partition wall **12**, the methane conversion rate can be stably improved.

The first end portions **2a** of the catalyst layers **2** are typically exposed on the first end surface **1a** of the honeycomb-like base material **1**, and the second end portions **2b** of the catalyst layers 2 are typically exposed on the second end surface **1b** of the honeycomb-like base material **1**.

As described above, the catalyst layers **2** contain the methanation reaction catalyst.

The methanation reaction catalyst typically contains an active component and a carrier that supports the active component.

The active component can promote the methanation reaction expressed by Formula (1) described above. An example of the active component is a metal element. More specific examples thereof include: alkali metal elements, such as K and Na; platinum group elements, such as Ir, Ru, and Rh; alkaline earth metal element such as Ca; and Ni. The metal elements may be incorporated in the methanation reaction catalyst alone or in combination thereof. Those metal elements may be incorporated in the methanation reaction catalyst in a metal state, may be incorporated in the methanation reaction catalyst as a metal salt, or may be incorporated in the methanation reaction catalyst as an oxide.

Of those metal elements, Ni is preferred, and Ni in a metallic state is more preferred.

An example of the carrier is a metal oxide. More specific examples thereof include: oxides of rare earth elements, such as cerium oxide and yttrium oxide; zirconium oxide; aluminum oxide; silicon oxide; and magnesium oxide. The carriers may be incorporated in the methanation reaction catalyst alone or in combination thereof. Of those carriers, oxides of rare earth elements are preferred, and cerium oxide is more preferred.

A content ratio of the active component is, for example, from 0.01 part by mass to 50 parts by mass, preferably from 1 part by mass to 20 parts by mass with respect to 100 parts by mass of the carrier. When the content ratio of the active component falls within such ranges, the methanation reaction can be stably promoted.

The methanation reaction catalyst may have any appropriate shape. The methanation reaction catalyst typically has a particulate shape. A particulate methanation reaction catalyst is hereinafter sometimes referred to as "catalyst particle". In one embodiment, the catalyst layers **2** each contain an aggregate in which a plurality of catalyst particles are gathered together. The aggregate of the plurality of catalyst particles may form mesopores in the catalyst layers **2**. The catalyst layers **2** may contain another component (for example, a binder) in addition to the methanation reaction catalyst. Typical examples of the binder include an inorganic binder. More specific examples thereof include $Al_2O_3$, $SiO_2$, and $ZrO_2$.

The average pore diameter in the catalyst layer **2** is, for example, from 0.2 $\mu$m to 10 $\mu$m, preferably from 1.0 $\mu$m to 5.0 $\mu$m.

The BET specific surface area of the catalyst layer **2** is, for example, from 10 $m^2$/g to 300 $m^2$/g, preferably from 50 $m^2$/g to 200 $m^2$/g, more preferably from 80 $m^2$/g to 150 $m^2$/g.

D. Method of Manufacturing Honeycomb Structural Body

Next, a method of manufacturing the honeycomb structural body **100** is described. In one embodiment, the method of manufacturing the honeycomb structural body **100** includes: a step of preparing the honeycomb-like base material **1;** and a step of forming the catalyst layers **2** on the partition wall **12** of the honeycomb-like base material **1.**

The honeycomb-like base material **1** is produced, for example, by the following method. First, a binder and water or an organic solvent are added to material powder containing the ceramic material described above, as required. The resultant mixture is kneaded to provide a body, and the body is molded (typically extruded) into a desired shape. After that, the body is dried, and is then fired as required.

Next, the catalyst layers **2** are formed on the partition wall **12**. A method of forming the catalyst layers **2** is not particularly limited, and any appropriate method may be adopted. In one embodiment, a step of forming the catalyst layers **2** includes: a step of preparing the catalyst particles; a step of preparing a catalyst slurry in which the catalyst particles are dispersed; and a step of applying the catalyst slurry onto the surface of the partition wall **12,** in the stated order.

In the step of preparing the catalyst particles, for example, a carrier dispersion liquid in which particles of the above-mentioned metal oxide (carrier) (hereinafter referred to as "metal oxide particles") are dispersed and a metal salt solution in which a salt of the above-mentioned metal element (active component) (hereinafter referred to as "metal salt") is dissolved are prepared.

For the preparation of the carrier dispersion liquid, the metal oxide particles are added to a dispersion medium and are agitated.

A primary particle diameter of the metal oxide particles is, for example, from 5 nm to 200 nm.

The amount of metal oxide particles to be added is, for example, from 0.5 part by mass to 25 parts by mass with respect to 100 parts by mass of the dispersion medium.

Any appropriate solvent in which the metal oxide particles are insoluble may be adopted as the dispersion medium. Examples of the dispersion medium include water and alcohols. The dispersion media may be used alone or in combination thereof. Of the dispersion media, water is preferred.

For the preparation of the metal salt solution, the metal salt is added to a solvent and is agitated.

Examples of the metal salt include a nitric acid salt, a chloride, and a bromide. Of those, a nitric acid salt is preferred, and nickel nitride is more preferred. The metal salts may be used alone or in combination thereof.

The amount of the metal salt to be added is, for example, from 1.0 part by mass to 30 parts by mass with respect to 100 parts by mass of the solvent.

Examples of the solvent include solvents similar to the dispersion media described above. The solvents can be used alone or in combination thereof. Of the solvents, the same solvent as the dispersion medium used for the carrier dispersion liquid is preferred.

Next, the metal salt solution is added to the carrier dispersion liquid and is agitated. As a result, a mixed liquid of the carrier dispersion liquid and the metal salt solution is prepared.

A mixture ratio of the carrier dispersion liquid and the metal salt solution may be appropriately and suitably adjusted so that the content ratio of the metal element (active component) in the methanation reaction catalyst to be produced falls within the above-mentioned ranges.

Next, the mixed liquid is heated while being agitated so that the dispersion medium and the solvent are evaporated. As a result, a solid is obtained. After that, the solid is heated.

A heating temperature for the solid is, for example, from 300°C to 700°C, preferably from 400°C to 600°C.

Heating time for the solid is, for example, from 0.5 hour to 8 hours, preferably from 2 hours to 4 hours.

The solid may be heated in the atmosphere or under a reducing atmosphere (typically, a hydrogen atmosphere). The solid is preferably heated in the atmosphere.

In this manner, methanation reaction catalyst particles (catalyst particles) are prepared.

A primary particle diameter of the catalyst particles is, for example, from 5 nm to 80 nm.

In the step of preparing the catalyst slurry, the catalyst particles are added to the dispersion medium and are agitated.

As the dispersion medium, any appropriate solvent in which the catalyst particles are insoluble may be adopted. Examples of the dispersion medium include water and alcohols. The dispersion media may be used alone or in combination thereof. Of the dispersion media, water is preferred.

In this manner, the catalyst particles are dispersed in the dispersion medium to thereby prepare the catalyst slurry.

A content ratio of the catalyst particles in the catalyst slurry is, for example, from 3.0 mass% to 30 mass%, preferably from 5.0 mass% to 20 mass%. When the content ratio of the catalyst particles in the catalyst slurry falls within such ranges, the catalyst slurry can be smoothly applied onto the partition wall and thus the catalyst layers can be stably formed.

Next, in the step of applying the catalyst slurry onto the surface of the partition wall **12,** the catalyst slurry is applied onto the partition wall **12** of the honeycomb-like base material **1** by any appropriate method.

In one embodiment, the honeycomb-like base material **1** is immersed into the catalyst slurry a plurality of times while changing a portion of the honeycomb-like base material **1** to be immersed.

For example, first, the entirety of the honeycomb-like base material **1** is immersed into the catalyst slurry. After that, the honeycomb-like base material **1** is removed from the catalyst slurry. In this manner, the catalyst slurry is applied onto the surface of the partition wall **12.** After that, coating films of the catalyst slurry formed on the partition wall **12** are dried at any appropriate heating temperature as required. In this manner, first layers are formed on the surface of the partition wall **12.**

Next, the honeycomb-like base material **1** in which the first layers are formed is moved in the lengthwise direction of the honeycomb-like base material **1** so as to be immersed into the catalyst slurry from the second end surface **1b** to any appropriate intermediate position (specifically, a position between the first end surface **1a** and the second end surface **1b**). After that, the honeycomb-like base material **1** is removed from the catalyst slurry. In this manner, the catalyst slurry is applied onto part of the first layers, which corresponds to the thick portions **22,** without being applied onto part of the first layers which corresponds to the thin portions **21.** After that, coating films of the catalyst slurry formed on the first layers are dried at any appropriate heating temperature as required. In this manner, second layers are formed on surfaces of the first layers. Further, the above-mentioned immersion and drying are repeated as required.

Through the above-mentioned process, the catalyst layers, each including the thin portion **21** and the thick portion **22,** can be formed on the partition wall **12.**

In another embodiment, the honeycomb-like base material **1** is immersed into the catalyst slurry a plurality of times while being kept in the same orientation.

For example, first, the honeycomb-like base material **1** is moved in the lengthwise direction thereof so as to be immersed into the catalyst slurry from the second end surface **1b** to the first end surface **1a.** After that, the honeycomb-like base material **1** is removed from the catalyst slurry. In this manner, the catalyst slurry is applied onto the surface of the partition wall **12.** Next, typically, the honeycomb-like base material **1** is arranged such that the second end surface **1b** of the honeycomb-like base material **1** faces downward and the first end surface **1a** of the honeycomb-like base material **1** faces upward, and is dried at any appropriate heating temperature. In this manner, the first layers are formed on the surface of the partition wall **12.** The first layers may each have a thickness increasing from the first end surface **1a** toward the second end surface **1b.**

Next, the honeycomb-like base material **1** in which the first layers are formed is moved in the lengthwise direction thereof, and is immersed into the catalyst slurry again from the second end surface **1b** to the first end surface **1a.** After that, typically, drying is performed in the same manner as that described above to thereby form the second layers on the surfaces of the first layers. Further, the above-mentioned immersion and drying are repeated as required.

Through the above-mentioned process, the catalyst layers **2,** each having a thickness continuously increasing from the first end surface **1a** toward the second end surface **1b,** can be formed on the partition wall **12.**

Further, as illustrated in FIG. **3,** a plurality of honeycomb-like base materials **1** may be prepared, and the catalyst slurry may be applied onto the partition walls **12** of the plurality of the honeycomb-like base materials **1** by any appropriate method. The thickness of each of the catalyst layers **2** formed on the partition wall **12** of each of the honeycomb-like base materials **1** can be adjusted by appropriately changing the number of repetitions of the application and the drying of the catalyst slurry. In this manner, the first block **3a** in which the thin portions **21** are formed on the partition wall **12** and the second block **3b** in which the thick portions **22** are formed on the partition wall **12** can be prepared separately.

Through such steps, the honeycomb structural body **100** including the honeycomb-like base material **1** and the catalyst layers **2** is manufactured.

The honeycomb structural body **100** is subjected to a reduction treatment as required. The reduction treatment allows the active component contained in the catalyst layers to be reduced to a metallic state. In the reduction treatment, the honeycomb structural body is thermally treated under an $H_2$ atmosphere. A heating temperature is, for example, from 300°C to 600°C, preferably from 400°C to 500°C. Heating time is, for example, from 0.1 hour to 8 hours, preferably from 0.5 hour to 2 hours.

E. Methane Production Apparatus and Method of Producing Methane

The above-mentioned honeycomb structural body **100** is a honeycomb structural body for a methanation reaction, and may suitably be used for a methane production apparatus that produces methane through the reaction between carbon dioxide and hydrogen.

The methane production apparatus includes at least the honeycomb structural body **100** described above. The methane production apparatus may include any appropriate component in addition to the honeycomb structural body **100.**

The methane production apparatus including the honeycomb structural body **100** can produce methane by supplying the raw material gas containing carbon dioxide and hydrogen to the catalyst layers **2** of the honeycomb structural body **100.**

In one embodiment, the honeycomb structural body **100** is heated to a predetermined methanation reaction starting temperature, and the raw material gas containing carbon dioxide and hydrogen is supplied to the gas flow passages **14** of the honeycomb structural body **100.** The methanation reaction starting temperature is, for example, from 200°C to 300°C.

As a result, the raw material gas is supplied to the catalyst layers **2** having the methanation reaction starting temperature to thereby start the methanation reaction expressed by Formula (1) described above.

The content ratio of carbon dioxide in the raw material gas is, for example, from 1.0 vol% to 20 vol%, preferably from 5.0 vol% to 15 vol%.

The content ratio of hydrogen in the raw material gas is, for example, from 4.0 vol% to 80 vol%, preferably from 20 vol% to 60 vol%.

In one embodiment, the raw material gas contains oxygen in addition to carbon dioxide and hydrogen. When the raw material gas contains oxygen, the combustion reaction expressed by the above-mentioned Formula (2) can progress upon contact of hydrogen and oxygen with the above-mentioned methanation reaction catalyst.

A content ratio of oxygen in the raw material gas is, for example, from 0.5 vol% to 10 vol%, preferably from 1.0 vol% to 5.0 vol%.

Further, the raw material gas may contain nitrogen as a residual part.

For a flow rate of such raw material gas, any appropriate value may be adopted. The flow rate of the raw material gas is, for example, from 500 mL/minute to 15,000 mL/minute.

When the raw material gas is supplied to the catalyst layers **2** having the methanation reaction starting temperature, and the methanation reaction expressed by the above-mentioned Formula (1) starts, the methanation reaction can be continued by using the reaction heat. Thus, the heating of the honeycomb structural body **100** from outside may be stopped. Under a state in which the heating of the honeycomb structural body **100** from outside is stopped, the honeycomb structural body **100** can be maintained at, for example, from 300°C to 500°C with the reaction heat. That is, the use of the honeycomb structural body **100** enables auto-methanation. Thus, energy savings in the production of methane can be achieved. Further, the honeycomb structural body **100** can be maintained at a temperature suitable for the methanation reaction without providing a heat retaining material to the honeycomb structural body. Thus, the methane production apparatus can be designed compactly.

As a result of the methanation reaction, a methane-containing gas is continuously discharged through the gas flow passages **14** of the honeycomb structural body **100.**

The methane-containing gas contains at least methane. The methane-containing gas may contain a residual unreacted raw material gas.

In such production of methane, the catalyst layers **2** have a difference in the supported catalyst amount described above. Thus, methane can be produced at an excellent methane conversion rate.

The methane conversion rate is, for example, 65% or more, preferably 70% or more, more preferably 75% or more, still more preferably 80% or more. Meanwhile, the methane conversion rate is, for example, 100% or less, and is, for example, 90% or less.

Examples

Now, the present disclosure is specifically described by way of Examples. However, the present disclosure is not limited by these Examples.

<Example 1>

<<Preparation of Honeycomb-like Base Material>>

After a body containing cordierite was subjected to extrusion, the body was dried to prepare the honeycomb-like base material illustrated in FIG. **1.** The honeycomb-like base material had a columnar shape with a diameter of 20 nm and a length of 50 mm. The honeycomb-like base material included: the partition wall that defined the plurality of cells; and the outer peripheral wall that surrounded the partition wall. The sectional shapes of the cells were each a quadrangle. The cell density of the honeycomb-like base material was 300 cpsi, and the thickness of the partition wall was 0.0254 mm. The mean pore diameter of the partition wall was 20 $\mu$m, and the porosity of the partition wall was 50%.

<<Preparation of Methanation Reaction Catalyst>>

After cerium oxide (IV) particles (manufactured by Daiichi Kigenso Kagaku Kogyo Co., Ltd) were introduced into distilled water, the distilled water was agitated under reduced pressure at room temperature (23°C) for 12 hours. In this manner, a metal-oxide-particle dispersion liquid was obtained. Further, a hexahydrate of nickel nitrate (II) was dissolved in distilled water to obtain a nickel nitrate aqueous solution. Next, the nickel nitrate aqueous solution was added to the metal-oxide-particle dispersion liquid, and was agitated at room temperature (23°C) for 2 hours. After that, a mixed liquid of the dispersion liquid and the aqueous solution was heated to 80°C while being agitated, to evaporate water. Next, a residual solid was heated at 500°C for 3 hours. In this manner, methanation reaction catalyst particles (hereinafter referred to as "catalyst particles") were obtained. The catalyst particles contained nickel oxide (NiO) and cerium oxide (IV) that supported nickel oxide (NiO). A Ni content in the methanation reaction catalyst particles was 10 parts by mass with respect to 100 parts by mass of cerium oxide.

<<Preparation of Catalyst Layers>>

A catalyst slurry was prepared by dispersing the resultant catalyst particles in distilled water. The content ratio of the catalyst particles in the catalyst slurry was 10 mass%. Next, the entirety of the honeycomb-like base material prepared as described above was immersed in the catalyst slurry under normal pressure (0.1 MPa) at room temperature (23°C) for 5 seconds. After that, the honeycomb-like base material was removed from the catalyst slurry. In this manner, the catalyst slurry was applied onto the surface of the partition wall. After that, the catalyst slurry applied onto the surface of the partition wall was heated at 100°C for 120 minutes to be dried. The above-mentioned immersion and drying were repeated three times. In this manner, the first layers were formed on the surface of the partition wall.

Next, the honeycomb-like base material in which the first layers had been formed was arranged such that the lengthwise direction of the honeycomb-like base material was substantially parallel to a vertical direction and the second end surface faced a liquid surface of the catalyst slurry. After that, the honeycomb-like base material was moved downward, and the honeycomb-like base material was immersed into the catalyst slurry from the second end surface to a predetermined intermediate position under the above-mentioned conditions for five seconds. Under a state in which the honeycomb-like base material was immersed into the catalyst slurry up to the intermediate position, the first end surface of the honeycomb-like base material was positioned away from the liquid surface of the catalyst slurry in the vertical direction.

After that, the honeycomb-like base material was removed from the catalyst slurry. In this manner, the catalyst slurry was applied onto the surfaces of the first layers. After that, the catalyst slurry that had been applied onto the surfaces of the first layers was heated at 100°C for 120 minutes to be dried. The above-mentioned immersion and drying were repeated seven times. In this manner, the second layers were formed on the surfaces of the first layers.

Through the above-mentioned process, the catalyst layers, each including the thin portion (first layer) and the thick portion (first layer and second layer), were formed. The catalyst layers each contained the aggregate of the catalyst particles.

When the overall length of the catalyst layer was defined as 100%, and the first end portion of the catalyst layer was defined as 0%, the thin portion was provided over the range of from 0% to 10% of the catalyst layer, and the thick portion was provided over the range of from 10% to 100% of the catalyst layer.

The thickness of the catalyst layer at the position corresponding to 5% (that is, the thin portion), the thickness of the catalyst layer at the position corresponding to 50% (that is, the thick portion), and the supported catalyst amounts in the thin portions and the thick portions, respectively, are shown in Table 1.

Through the steps described above, the honeycomb structural body including the honeycomb-like base material and the catalyst layers was formed.

<Comparative Example 1>

The honeycomb-like base material and the catalyst slurry were prepared in the same manner as in Example 1. After that, the honeycomb-like base material was arranged such that the lengthwise direction of the honeycomb-like base material was substantially parallel to the vertical direction and the second end surface faced the liquid surface of the catalyst slurry. After that, the honeycomb-like base material was moved downward, and the honeycomb-like base material was immersed into the catalyst slurry from the second end surface to the first end surface of the honeycomb-like base material in the same manner as that described above. After that, the honeycomb-like base material was removed from the catalyst slurry. In this manner, the catalyst slurry was applied onto the surface of the partition wall. After that, the catalyst slurry that had been applied onto the surface of the partition wall was heated in the same manner as that described above to be dried. In this manner, the first layers were formed on the surface of the partition wall.

Next, an orientation of the honeycomb-like base material in which the first layers had been formed was reversed, and the honeycomb-like base material was arranged such that the first end surface faced the liquid surface of the catalyst slurry. After that, the honeycomb-like base material was moved downward, and the honeycomb-like base material was immersed into the catalyst slurry from the first end surface to the second end surface of the honeycomb-like base material in the same manner as that described above. After that, the honeycomb-like base material was removed from the catalyst slurry. In this manner, the catalyst slurry was applied onto the surfaces of the first layers. After that, the catalyst slurry that had been applied onto the surfaces of the first layers was heated in the same manner as that described above to be dried. In this manner, the second layers were formed on the surfaces of the first layers. The above-mentioned immersion and drying were repeated seven times.

Through the above-mentioned process, the catalyst layers, each having a substantially uniform thickness throughout its entirety, were formed to thereby form the honeycomb structural body. The thickness of the catalyst layer at the position corresponding to 5%, the thickness of the catalyst layer at the position corresponding to 50%, and the supported catalyst amounts are shown in Table 1.

<Auto-methanation Test>

The honeycomb structural bodies obtained in Example and Comparative Example were each inserted into a reaction tube with an inner diameter of 21 mm. As a pre-treatment for the reaction, the honeycomb structural body was heated to 500°C by an electric furnace that was installed on an outer periphery of the reaction tube, and a hydrogen gas was introduced into the reaction tube to perform a reduction treatment. After that, a temperature of the electric furnace was lowered to 200°C, and a mixed gas of carbon dioxide at 10 vol%, oxygen at 3 vol%, hydrogen at 46 vol%, and nitrogen for a residual part was introduced as a raw material gas into the reaction tube. The flow rate of the raw material gas introduced into the reaction tube was set to 1,000 mL/minute. As a result, the raw material gas passed through the gas flow passages of the honeycomb structural body, and a methane-containing gas flowed out from the reaction tube.

The set temperature of the electric furnace was gradually lowered while the raw material gas was kept flowing through the reaction tube, and eventually, the heating by the electric furnace was stopped. A temperature at the first end portion of the catalyst layer after the stop of heating by the electric furnace was measured with a thermocouple. The results thereof are shown in Table 1.

A concentration of carbon dioxide and a concentration of methane in the methane-containing gas that had flowed out from the reaction tube after the stop of the heating by the electric furnace were measured with a gas chromatography-thermal conductivity detector (GC-TCD). Based on the results of the measurement, a conversion rate (%) of carbon dioxide to be converted to methane was calculated by the following Equation (I).

Methane conversion rate (%)= (amount of methane (vol%) contained in methane-containing gas/amount of carbon dioxide (vol%) contained in raw material gas)×100     (I)

[Table 1]

| No. | Average thickness of catalyst layer [μm] | | Supported amount of catalyst particles [g/mm²] | | Temperature at first end portion of catalyst layer [°C] | Methane conversion rate [%] |
|---|---|---|---|---|---|---|
| | Position corresponding to 5% | Position corresponding to 50% | Thin portion | Thick portion | | |
| Example 1 | 10 | 30 | 3.0 | 6.0 | 407 | 76 |
| Comparative Example 1 | 20 | 20 | 4.0 | | 430 | 68 |

<Evaluation>

As shown in Table 1, it is understood that, when the catalyst layers have a difference in the supported amount of methanation reaction catalyst in the direction of passage of the fluid through the gas flow passages (in particular, when the supported amount in the upstream-side part is relatively small, and the supported amount in the downstream-side part is relatively large), the methane conversion rate can be markedly improved.

Industrial Applicability

The honeycomb structural body according to the embodiments of the present disclosure can be suitably used for a methane production apparatus that produces methane through the reaction between carbon dioxide and hydrogen.

Reference Signs List

1    honeycomb-like base material
12   partition wall
13   cell
14   gas flow passage
2    catalyst layer
2a   first end portion of catalyst layer
2b   second end portion of catalyst layer
21   thin portion
22   thick portion
100  honeycomb structural body

**Claims**

1.  A honeycomb structural body, comprising:

    a honeycomb-like base material including a partition wall that defines a plurality of cells, each including a gas flow passage; and
    catalyst layers containing a methanation reaction catalyst for methanation of carbon dioxide through a reaction with hydrogen, the catalyst layers being provided on a surface of the partition wall so as to face the gas flow passages, respectively,
    wherein the catalyst layers have a difference in the amount of the methanation reaction catalyst supported per unit area of the partition wall in a direction of passage of a fluid through the gas flow passages.

2.  The honeycomb structural body according to claim 1, wherein the supported amount of the methanation reaction catalyst increases toward a downstream side in the passage direction.

3.  The honeycomb structural body according to claim 2,

wherein the catalyst layers each have a first end portion being an upstream end portion in the passage direction, and a second end portion being a downstream end portion in the passage direction, and

wherein, when an overall length of each of the catalyst layers in the passage direction is defined as 100% and the first end portion of the catalyst layer is defined as 0%, the supported amount of the methanation reaction catalyst in a range of from 0% to 10% of the catalyst layer is smaller than the supported amount of the methanation reaction catalyst in a range of from 50% to 100% of the catalyst layer.

4. The honeycomb structural body according to claim 2, wherein, when an overall length of each of the catalyst layers in the passage direction is defined as 100% and a first end portion of the catalyst layer is defined as 0%, a thickness of the catalyst layer at a position corresponding to 50% is larger than a thickness of the catalyst layer at a position corresponding to 5%.

5. The honeycomb structural body according to claim 4, wherein the catalyst layers each include a thin portion and a thick portion in the stated order in the passage direction.

6. The honeycomb structural body according to any one of claims 1 to 5, wherein the methanation reaction catalyst contains Ni as an active component, and a cerium oxide carrier for supporting Ni.

FIG. 1

FIG. 2

FIG. 3

**EP 4 691 639 A1**

## INTERNATIONAL SEARCH REPORT

| | International application No. |
|---|---|
| | **PCT/JP2024/011126** |

**A. CLASSIFICATION OF SUBJECT MATTER**

*B01J 35/57*(2024.01)i; *B01J 23/80*(2006.01)i; *C07B 61/00*(2006.01)i; *C07C 1/12*(2006.01)i; *C07C 9/04*(2006.01)i
FI: B01J35/57 L; B01J23/80 M; C07C1/12; C07C9/04; C07B61/00 300

According to International Patent Classification (IPC) or to both national classification and IPC

**B. FIELDS SEARCHED**

Minimum documentation searched (classification system followed by classification symbols)

B01J35/57: B01J23/80; C07B61/00; C07C1/12; C07C9/04

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2005-538019 A (ENGELHARD CORPORATION) 15 December 2005 (2005-12-15) claims, paragraphs [0031], [0032], fig. 5 | 1-5 |
| Y | | 6 |
| Y | JP 2023-17750 A (PROTERIAL LTD.) 07 February 2023 (2023-02-07) claims, examples, fig. 1, 2 | 1-6 |
| Y | JP 2020-93216 A (IHI CORP.) 18 June 2020 (2020-06-18) claims, paragraphs [0033]-[0035], [0048], [0049] | 1-6 |
| Y | JP 2020-33280 A (NATIONAL UNIVERSITY CORP. SHIZUOKA UNIVERSITY) 05 March 2020 (2020-03-05) claims, examples | 6 |
| Y | JP 2019-155227 A (HITACHI CHEMICAL CO., LTD.) 19 September 2019 (2019-09-19) claims, examples | 6 |

☑ Further documents are listed in the continuation of Box C.　　☑ See patent family annex.

\* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **16 May 2024** | **28 May 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

## INTERNATIONAL SEARCH REPORT

| | International application No. |
|---|---|
| | **PCT/JP2024/011126** |

### C. DOCUMENTS CONSIDERED TO BE RELEVANT

| Category* | Citation of document, with indication, where appropriate, of the relevant passages | Relevant to claim No. |
|---|---|---|
| Y | WO 2021/045101 A1 (NATIONAL UNIVERSITY CORP. SHIZUOKA UNIVERSITY) 11 March 2021 (2021-03-11) claims, examples | 6 |

Form PCT/ISA/210 (second sheet) (July 2022)

INTERNATIONAL SEARCH REPORT
Information on patent family members

International application No.

**PCT/JP2024/011126**

| Patent document cited in search report | | | Publication date (day/month/year) | Patent family member(s) | | | Publication date (day/month/year) |
|---|---|---|---|---|---|---|---|
| JP | 2005-538019 | A | 15 December 2005 | US 2004/0048114 A1 claims, paragraphs [0060], [0061], fig. 5<br>WO 2004/022481 A1<br>KR 10-2005-0057179 A | | | |
| JP | 2023-17750 | A | 07 February 2023 | (Family: none) | | | |
| JP | 2020-93216 | A | 18 June 2020 | (Family: none) | | | |
| JP | 2020-33280 | A | 05 March 2020 | (Family: none) | | | |
| JP | 2019-155227 | A | 19 September 2019 | (Family: none) | | | |
| WO | 2021/045101 | A1 | 11 March 2021 | US 2022/0298086 A1 claims, examples<br>EP 4026822 A1<br>CN 114341083 A<br>KR 10-2022-0053579 A | | | |

Form PCT/ISA/210 (patent family annex) (July 2022)

19

## EP 4 691 639 A1

**Patent documents cited in the description**

- JP 2015196619 A